# EUROPÄISCHE PATENTSCHRIFT

(11) **EP 2 437 836 B1**
(45) Veröffentlichungstag und Bekanntmachung des Hinweises auf die Patenterteilung: **06.07.2016**
(21) Anmeldenummer: 10727645.3
(22) Anmeldetag: 18.05.2010
(51) Int. Cl.: A61M 16/06

(54) **VORRICHTUNG ZUR BEATMUNG MIT EINER BEFESTIGUNGSEINRICHTUNG**
RESPIRATORY DEVICE COMPRISING A FASTENING SYSTEM
APPAREIL D'ASSISTANCE RESPIRATOIRE À DISPOSITIF DE FIXATION

(30) Priorität: 18.05.2009 DE 102009021807
(43) Veröffentlichungstag der Anmeldung: 11.04.2012
(60) Teilanmeldung: 13005484.4
(73) Patentinhaber: Weinmann Geräte für Medizin GmbH + Co. KG, 22525 Hamburg (DE)
(72) Erfinder: EIFLER, Martin, 25348 Glückstadt (DE); HAHN, Henry, 22525 Hamburg (DE); GARDEIN, Joachim, E-38430 Icod de los Vinos (ES); WONSYLD, Anne, 20259 Hamburg (DE); FELDHAHN, Karl-Andreas, Dr., 22761 Hamburg (DE)
(74) Vertreter: Marx, Thomas
(86) Internationale Anmeldenummer: PCT/DE2010/000584
(87) Internationale Veröffentlichungsnummer: WO 2010/133218

(56) Entgegenhaltungen:
- EP-A2- 1 493 461
- EP-A2- 2 005 987
- WO-A1-2007/143793
- WO-A2-2004/021960
- WO-A2-2007/021777
- WO-A2-2008/036625
- AU-A1- 2007 221 773
- DE-C1- 10 057 893
- US-A1- 2007 062 537
- US-A1- 2007 240 721

## Beschreibung

Die Erfindung betrifft eine Vorrichtung zur Beatmung mit einer Befestigungseinrichtung zur Befestigung eines Patienten Interfaces am Kopf des Anwenders. Patienten Interfaces dienen dazu, von einem Beatmungsgerät, bereitgestelltes Atemgas an den Patienten abzugeben. Patienteninterfaces können in unterschiedlichen Ausführungsformen beispielsweise als Sauerstoffbrillen, Nasal Pillow-, Nasaloder Vollgesichtsmasken realisiert sein. Das Patienten Interface ist typischerweise über einen Atemgasschlauch mit dem Beatmungsgerät verbunden und wird am Kopf des Anwenders fixiert. Eine genaue Passform des Patienten Interfaces sowie der Befestigung ist erforderlich um eine TherapieBeeinträchtigung durch z.B. Verrutschen oder Lösen zu vermeiden. Zur Gewährleistung einer sicheren Positionierung der Atemmaske im Bereich des Gesichtes eines Patienten, sowie der Verringerung der Kräfte die auf das Gesicht wirken, werden Atemmasken mit Stirnabstützungen benutzt. Derartige Stirnstützen besitzen typischerweise Höhen- oder Abstandsverstellmöglichkeiten. Viele Verstelleinrichtungen sind für den Patienten aber oft zu kompliziert und sind meist nur aufwendig vom Patienten oder im Schlaflabor einstellbar. Eine direkte Anpassung beim Tragen der Maske ist meist nicht möglich.

Durch die zusätzliche Verwendung einer Stirnstütze an dem gaszuführenden Patienten Interface, wie z.B. einer Nasalmaske, wird die sichere Positionierung der Atemmaske im Bereich des Gesichtes eines Patienten gewährleistet. Ein ungewolltes Ablösen oder Verrutschen der Maske vom Gesicht des Benutzers und daraus folgende Leckagen werden vermieden. Das Verrutschen eines schlecht sitzenden Patienten Interfaces kann zu unangenehmen Druckstellen bis hin zu Therapiebeeinträchtigungen bzw. Therapieunterbrechungen führen. Sowohl der Tragekomfort als auch die Stabilität werden durch das Verwenden einer Stirnabstützung erhöht. Es lassen sich Druckstellen im Anlagebereich der Maske vermeiden, die bei einer Beatmungsmaske ohne Stirnabstützung durch die Vorspannung der Bänderüng entstehen würden.

WO 2008036625 A2 offenbart eine Beatmungsmaske mit einer zweiteiligen Stirnstützenverstellung. Ein beweglicher Stirnstützenträgerarm ist schwenkbar im Maskenkörper gelagert und ein feststehender Stirnstützenschaft taucht vorgebbar weit durch eine Aussparung des Stirnstützenträgerarmes hindurch. Im Bereich der Aussparung des Stirnstützenträgerarmes, an der \ Kontaktstelle zum Schaft sind mehrere Rastpositionen vorhanden, über die die Position des Stirnstützenträgerarmes festgelegt werden kann. Der Abstand der Stirnstütze wird vom Nutzer manuell durch Verschwenken und Einrasten des Stirnstützenträgerarmes festgelegt.

WO 2007143793 A1 offenbart eine Beatmungsmaske mit einer zweiteiligen Stirnstützenverstellung. Ein beweglicher rohrartiger Stirnstützenträgerarm taucht vorgebbar weit durch eine runde Aussparung des feststehenden Stirnstützenschaftes hindurch. Im dem vom Nutzer abgewandten Bereich der Aussparung des Stirnstützenschaftes wird ein drehbares Betätigungselement auf das rohrartige Ende des Stirnstützenträgerarm geschoben. Das Betätigungselement weist einen Zapfen mit Außengewinde, der in das rohrartige Ende des Stirnstützenträgerarmes eintaucht, und das rohrartigen Ende des Stirnstützenträgerarm ein korrespondierendes Innengewinde auf. Durch Drehen des Betätigungselementes, kann so- über die Gewinde - die Position des Stirnstützenträgerarmes schraubenartig verändert werden.

WO 2007021777 A2 offenbart eine Beatmungsmaske mit einer einteiligen Stirnstütze ohne Verstellmöglichkeit. Im dem vom Nutzer abgewandten Bereich des Maskenkörpers, an der Basis des Stirnstützenschaftes ist ein drehbares Betätigungselement angeordnet, das die Neigung des Maskenwulstes, der zur Abdichtung gegen das Gesicht vorgesehenen ist, verändert. Das Betätigungselement bewegt auch hier einen schraubenartigen Mechanismus, der die Neigung des Wulstes relativ zum Maskenkörper verändert.

AU 2007221773 A1 offenbart eine Beatmungsmaske mit einer zweiteiligen Stirnstützenverstellung. Ein Stirnstützenträgerarm weist eine gebogene Kontur auf -und taucht mit einem Teil der gebogenen Kontur in eine korrespondierende Aufnahme in den Stirnstützenschaft ein. Der Stirnstützenträgerarm wird so in der Aufnahme des Stirnstützenschaftes gelagert. Ein Federmechanismus drängt den Stirnstützenträgerarm in der Aufnahme des Stirnstützenschaftes gegen den Stirnstützenschaft und arretiert die Position dadurch. Ein Betätigungselement ist mit dem Federmechanismus verbunden. Wenn der Nutzer das Betätigungselement bedient entlastet der Federmechanismus die Kontaktstelle von Stirnstützenträgerarm zum Stirnstützenschaft, woraufhin die Position des Stirnstützenträgerarmes veränderbar ist.

US2007240721 A1 offenbart eine Beatmungsmaske mit einer Schlauchführung nach oben über den Kopf. Der Schlauch wird in Stirnhöhe über eine Schlauchkupplung an einem Stirnband befestigt. Hierbei wird eine Verstellung des Abstands und der Neigung des Schlauches relativ zum Stirnband offenbart. In einem Ausführungsbeispiel ist dem Schlauch ein Blatt angeformt, dass in einem Schlitz, der mit dem Stirnband verbunden ist, eintaucht und dort mittels einer Schraube fixierbar ist.

EP 1493461 A2 offenbart eine Beatmungsmaske mit einer mehrteiligen Stirnstützenverstellung. Ein beweglicher Stirnstützenträgerarm ist im Stirnstützenschaft gelagert. Dµer Stirnstützenschaft weist dazu eine Aussparung auf, in der eine Aufnahme für den Stirnstützenträgerarm vorgesehen ist. Der Stirnstützenträgerarm ragt durch die Aufnahme hindurch.
In diesem Bereich der Aufnahme sind Rastelemente vorgesehen, die in korrespondierende Rastaufnahmen im Stirnstützenträgerarm eingreifen. Durch Drücken von Betätigungselementen im Bereich der Aufnahme, kann die Verrastung gelöst werden und der Stirnstützenträgerarm in der Aufnahme bewegt werden.

WO2004021960 A2 offenbart eine Beatmungsmaske mit einer mehrteiligen Stirnstützenverstellung. Ein Stirnstützenträgerarm weist eine gebogene Kontur auf. Der Stirnstützenschaft weist ebenfalls eine gebogene Kontur auf und taucht in eine korrespondierende Aufnahme des Stirnstützenträgerarmes ein. Der Stirnstützenträgerarm weist dort zusätzlich zwei Betätigungselemente in Form von Federlaschen auf. Diese greifen in Aufnahmen des Stirnstützenschaftes und arretiert die Position des Stirnstützenträgers dadurch. Wenn der Nutzer die Betätigungselemente bedient, rastet der Federmechanismus aus, woraufhin die Position des Stirnstützenträgerarmes entlang der gebogenen Kontur veränderbar ist.

DE10057893 C1 offenbart eine Beatmungsmaske mit einer Schlauchführung nach oben über den Kopf. Der Schlauch wird in Stirnhöhe über eine Schlauchkupplung an einer Stirnstütze befestigt, wobei die Stirnstütze mittels einer Bänderung am Kopf befestigt ist. Hierbei wird eine Verstellung des Abstands und der Neigung des Schlauches relativ zur Stirnstütze offenbart. Ein Stirnstützenträger umgibt den und weist zwei Federlaschen als Bedienelemente auf, die in die Stirnstütze eintauchen und dort verrasten. Da in der Stirnstütze verschiedene Verrastpositionen vorhanden sind, lässt sich die Position der Stirnstütze relativ zum Schlauch verändern.

US 2007/062537 A1 offenbart eine Beatmungsmaske mit einer mehrteiligen Stirnstützenverstellung. Ein Stirnstützenträgerarm ist an zwei Punkten schwenkbar auf zwei Stirnstützenschäften gelagert. Die zwei Stirnstützerischäfte weisen eine unterschiedliche Länge auf und sind selbst ebenfalls schwenkbar im Maskenkörper gelagert. Eine Gewindestange verbindet die zwei Stirnstützenschäfte so dass die Gewindestange durch einen Schaft mit Gewindebohrung hindurch ragt. Über ein Betätigungselement im Bereich der Gewindestange kann diese gedreht werden, wobei ein Drehen der Gewindestange bewirkt, das ein Schaft bewegt wird und so ein Einstellen der Position der Stirnstütze möglich ist.

EP 2005987 A2 offenbart eine Beatmungsmaske mit einer zweiteiligen Stirnstützenverstellung. Ein beweglicher Stirnstützenträgerarm ist an einer Achse schwenkbar im Stirnstützenschaft gelagert. Im dem vom Nutzer abgewandten Bereich des Stirnstützenschaftes weist dieser eine Aussparung auf, durch die ein Betätigungselement Stirnstützenträgerarmes hindurch ragt. In diesem Bereich weisen Stirnstützenträgerarm und -Schaft korrespondierende Rastelemente auf. Durch Drücken des Betätigungselementes, kann so die Verrastung gelöst werden und der Stirnstützenträgerarm verschwenkt werden.

Nachteilig an dieser einteiligen Lösung ist, dass die Verrastung zunächst durch Drücken des Betätigungselementes gelöst werden muss und der Stirnstützenträgerarm nur bei gleichzeitigem Niederdrücken des Betätigungselementes verschwenkt werden kann. Die gerastete Verstellung ist zudem recht grobstufig.

Der Nutzer muss Einstellungen schnell und sicher umsetzen können und, in bestimmten Positionen, die Einstellungen sehr fein dosieren können, um eine optimale Anpassung der Stirnstütze an seine Gesichtsanatomie vornehmen zu können. Die Aufgabe der vorliegenden Erfindung ist es, dem Patienten bei gleichbleibend kompakter Bauform eine sichere und schnelle Verstellung der Stirnstütze zu ermöglichen und insbesondere eine Übersetzung der Bewegung des Stellgliedes, auf die stufenlose Bewegung der Stirnstütze zu realisieren.

Die Erfindung wird durch die Merkmale des unabhängigen Anspruchs 1 definiert. Erfindungsgemäß wird diese Aufgabe dadurch gelöst, dass die Vorrichtung zur Beatmung, zumindest aus einem Maskenkörper besteht, welcher einen Innenraum von einem Aussenbereich abgrenzt, einem Bereich zur Abdichtung der Vorrichtung gegen Gesichtspartien eines Patienten, Befestigungselementen zur Positionierung der Vorrichtung am Kopf des Patienten und einem Anschluss zur Zufuhr von Atemgas, sowie einer der Abstützung an der Stirn dienende Stirnstütze, die einen Stirnstützenträgerarm aufweist, wobei der Stirnstützenträgerarm Führungsnuten aufweist, wobei der Maskenkörper einen fest verbundenen Stirnstützenschaft aufweist, wobei die Verbindung zwischen Stirnstützenschaft und Stirnstützenträgerarm über zwei Halteelemente erfolgt, die am Stirnstützenträgerarm angeordnet und drehbar im Stirnstützenschaft gelagert sind dadurch gekennzeichnet, dass das Verstellen über das Bewegen eines als Schieber ausgebildeten Stellgliedes, in einer gestreckten Bewegung, erfolgt, wobei der Schieber über den feststehenden Stirnstützenschaft greift und wobei der Schieber Führungselemente aufweist, die in Führungsnuten im Stirnstützenträgerarm greifen und diesen beim Verstellen vor und zurück bewegen, wobei der Stirnstützenträgerarm schmaler als der Stirnstützenschaft ist.

Die Veränderung des Abstandes kann in unterschiedlichen Varianten erfolgen. Gemeinsam ist allen nachfolgend beschriebenen Beispielen , daß eine für den Patienten einfache Bedienung erfolgen kann, eine hohe Robustheit der Vorrichtung erreicht ist und trotzdem eine unbeabsichtigte Verstellung vermieden wird.

In den Zeichnungen sind Beispiele schematisch dargestellt.

Es zeigen:

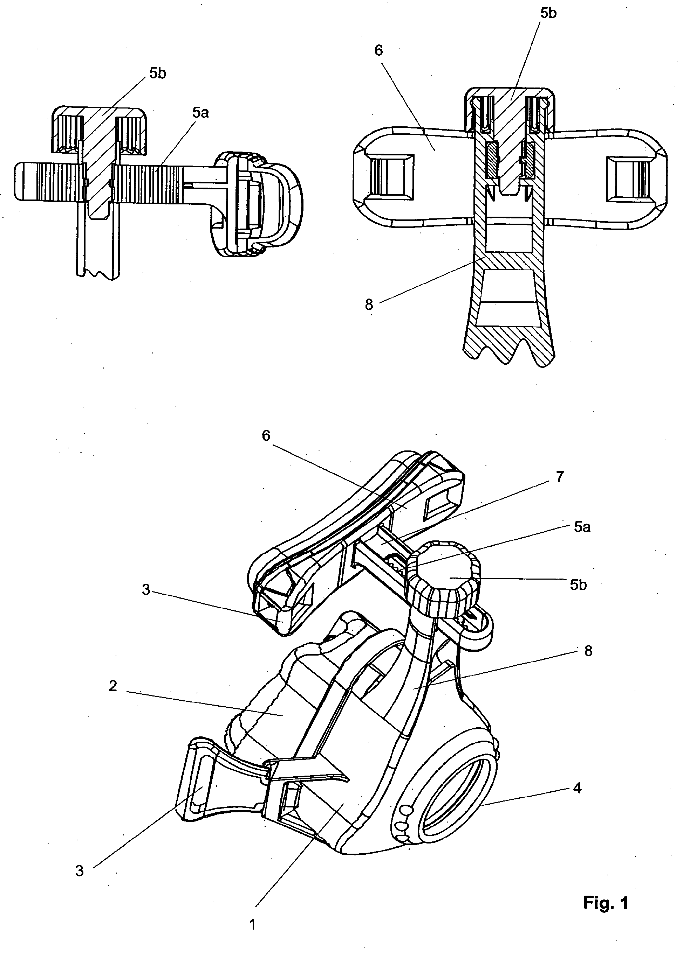

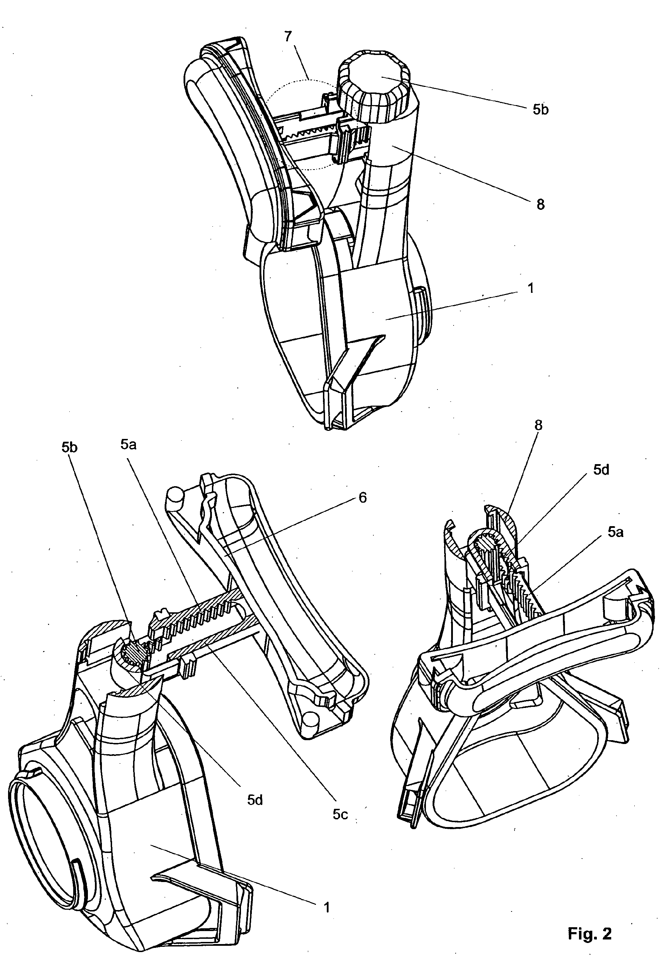

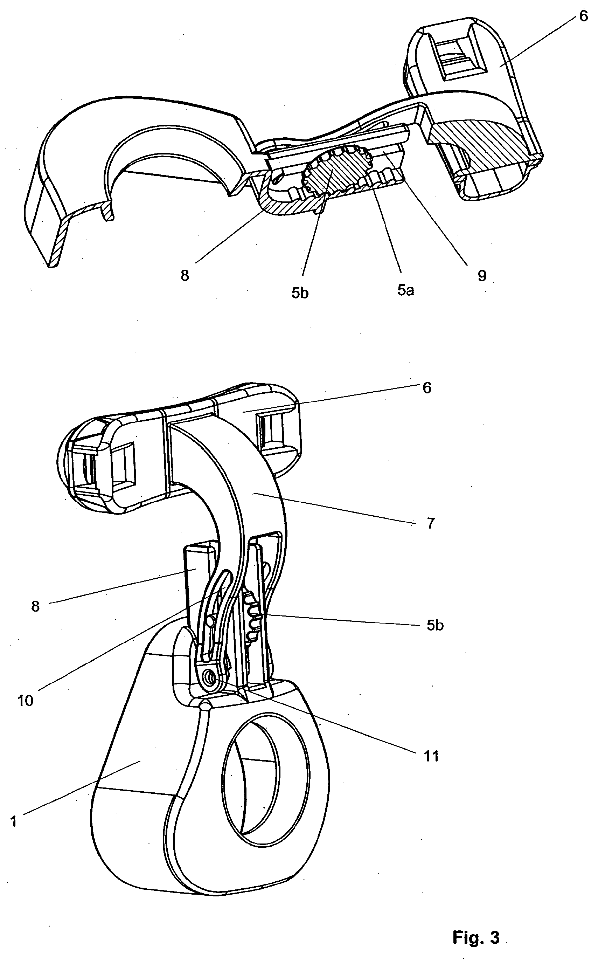

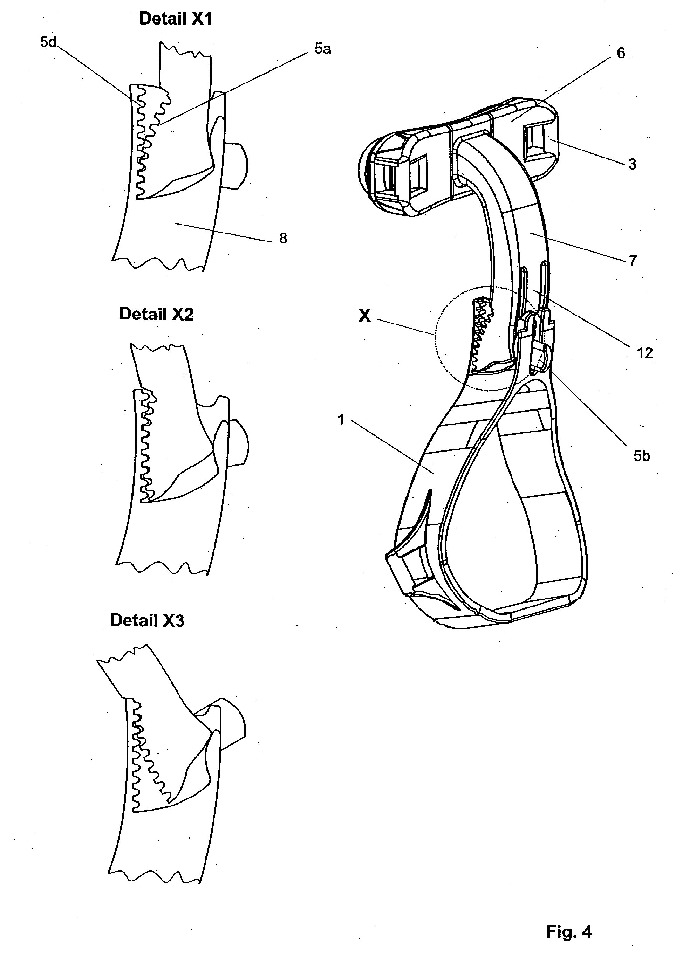

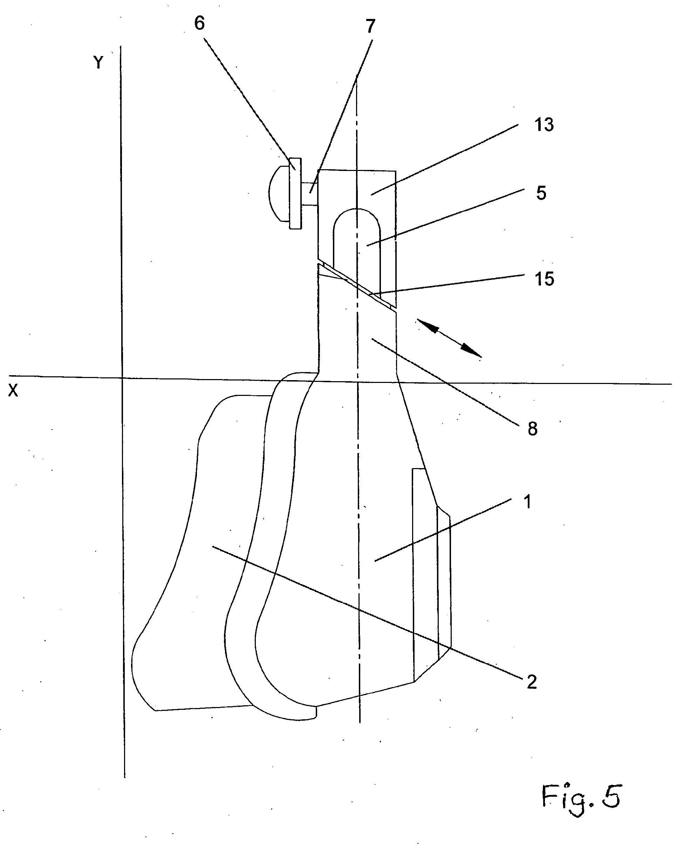

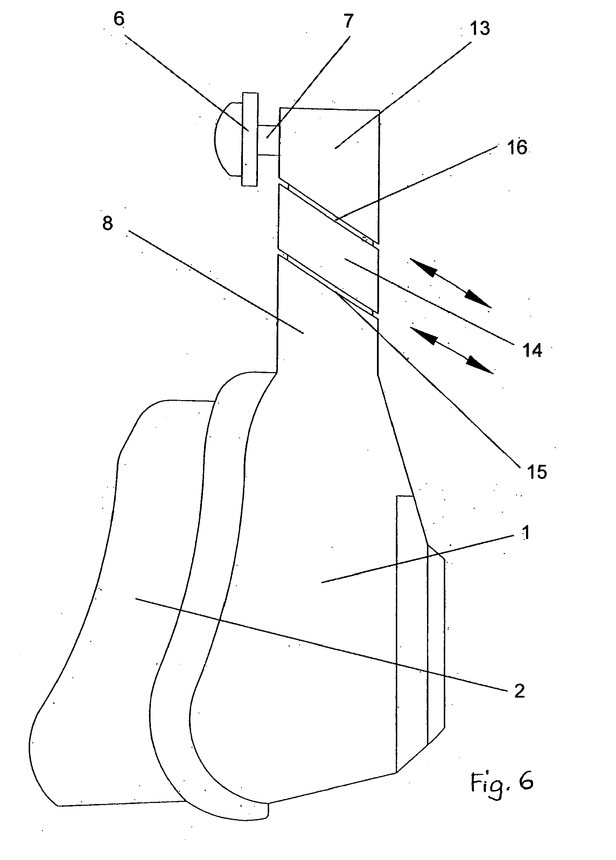

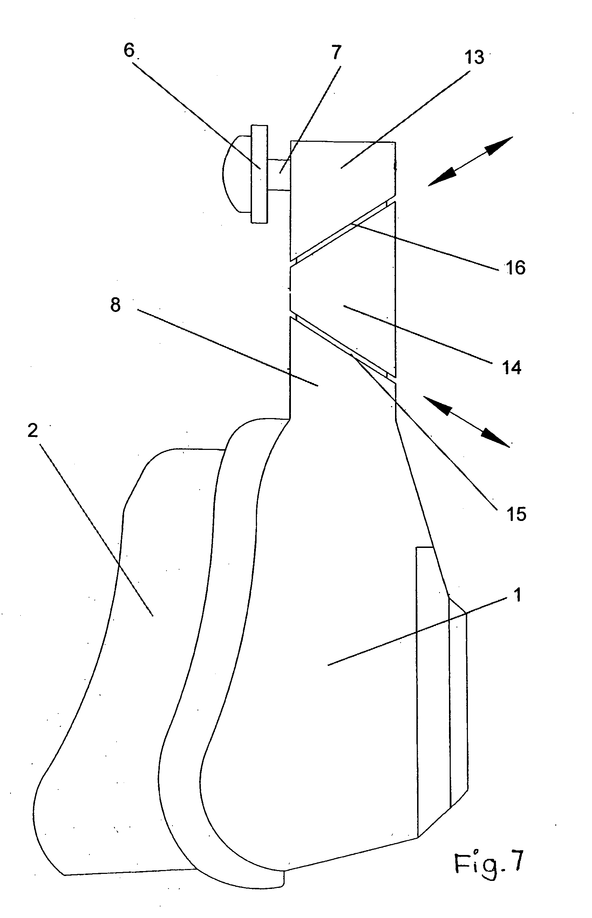

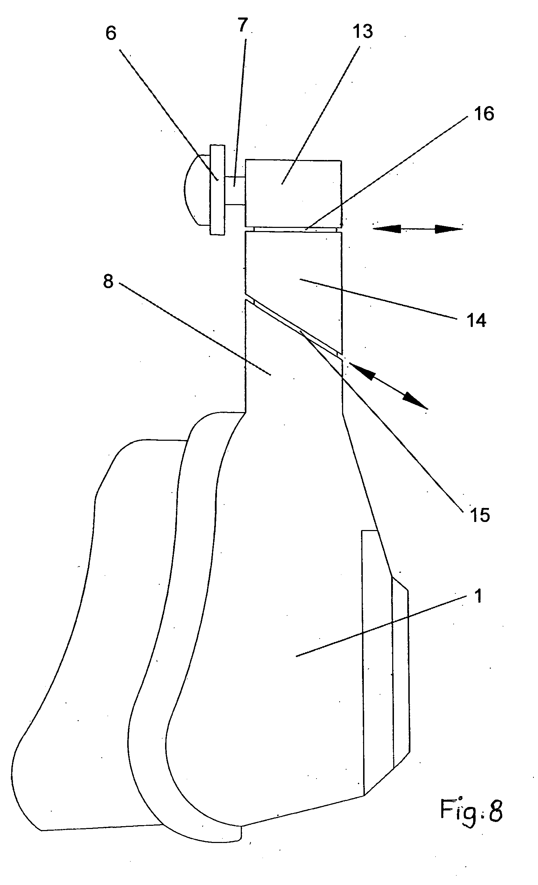

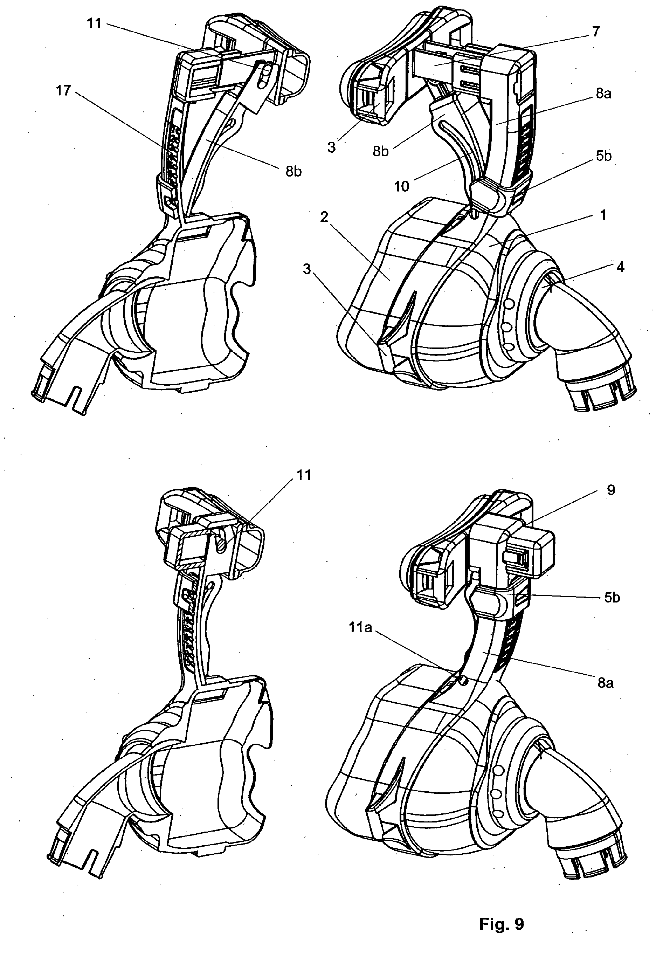

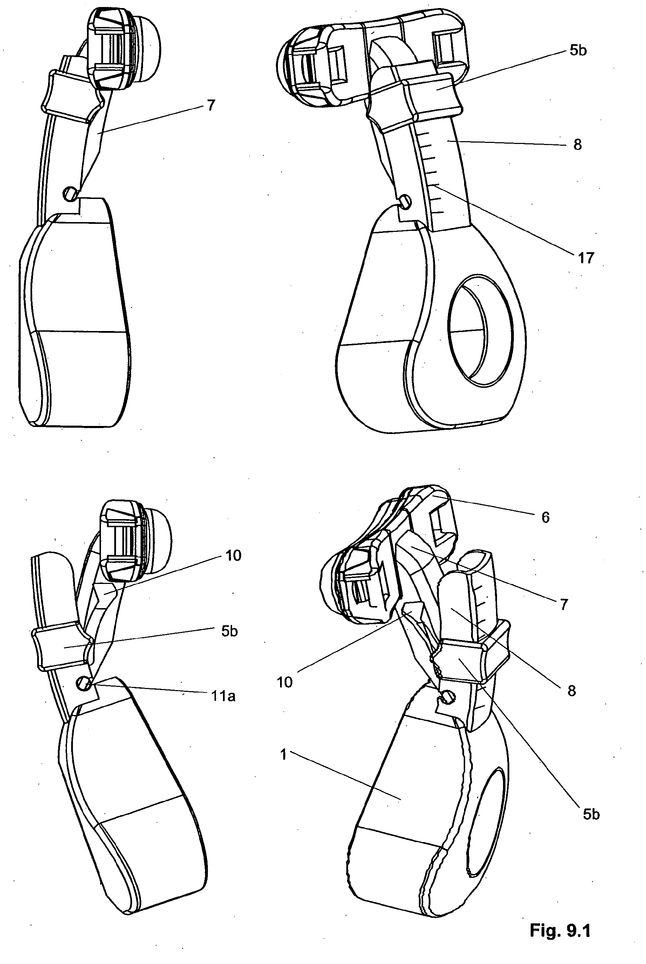

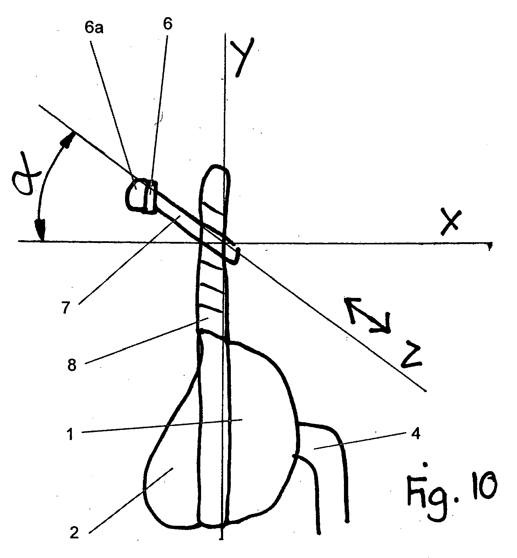

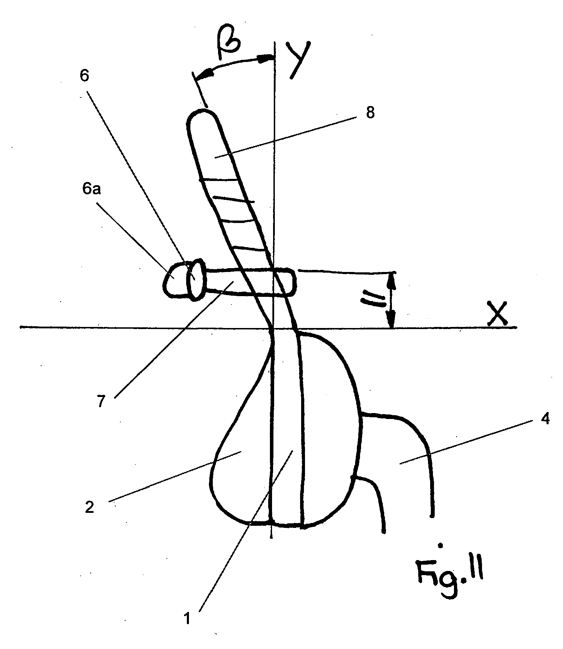

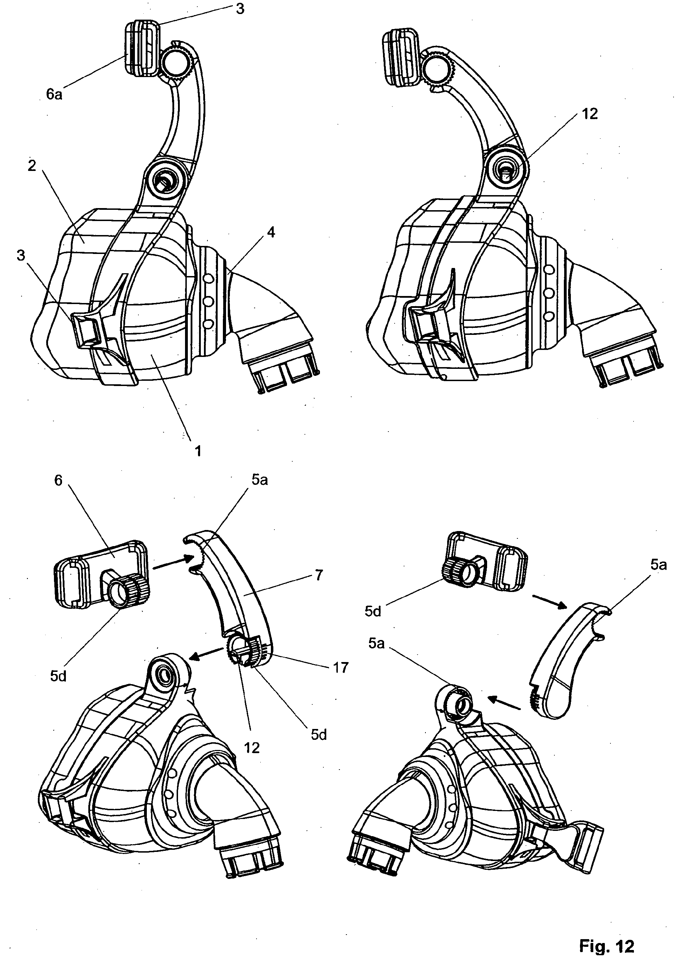

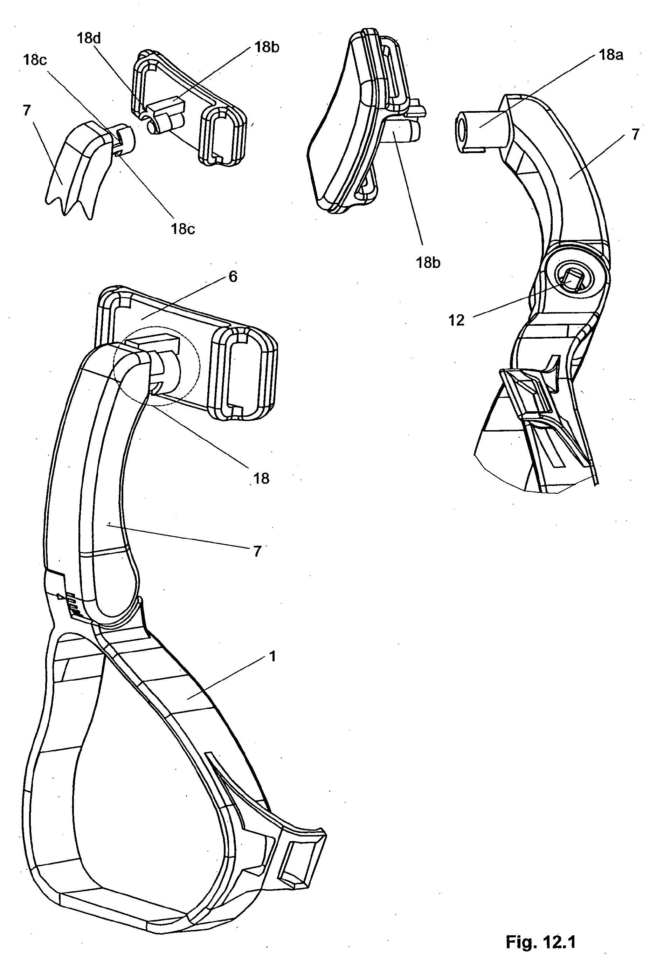

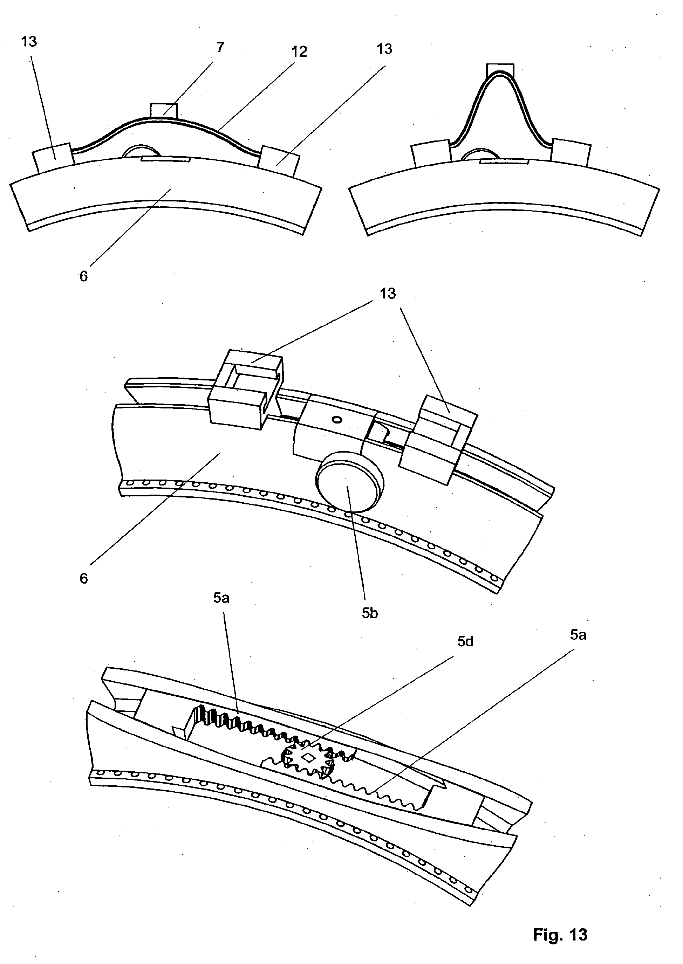

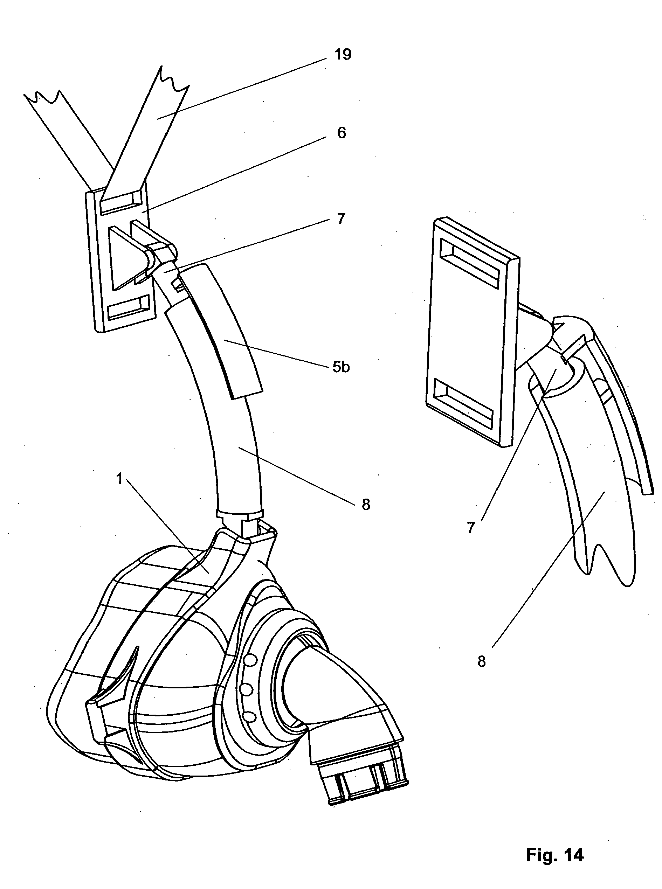

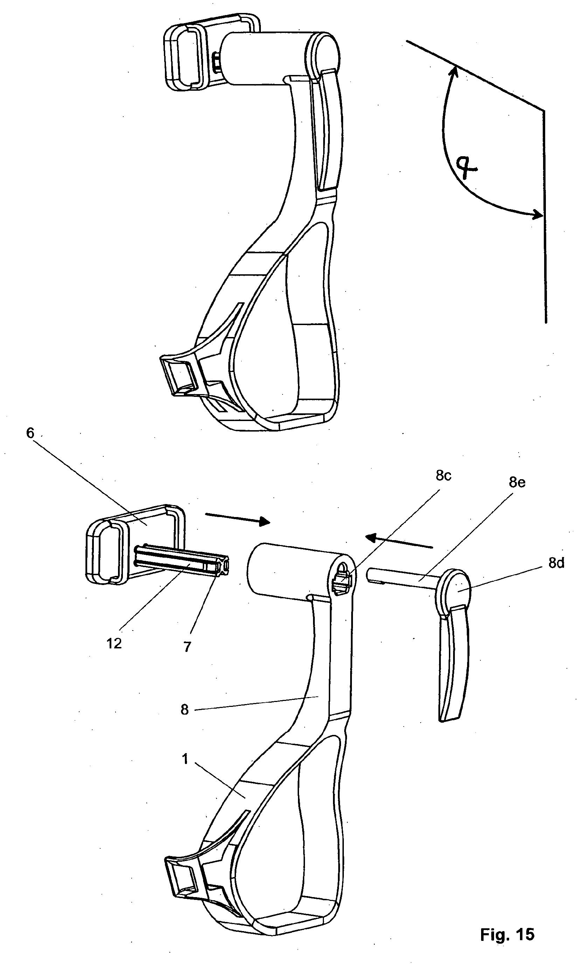

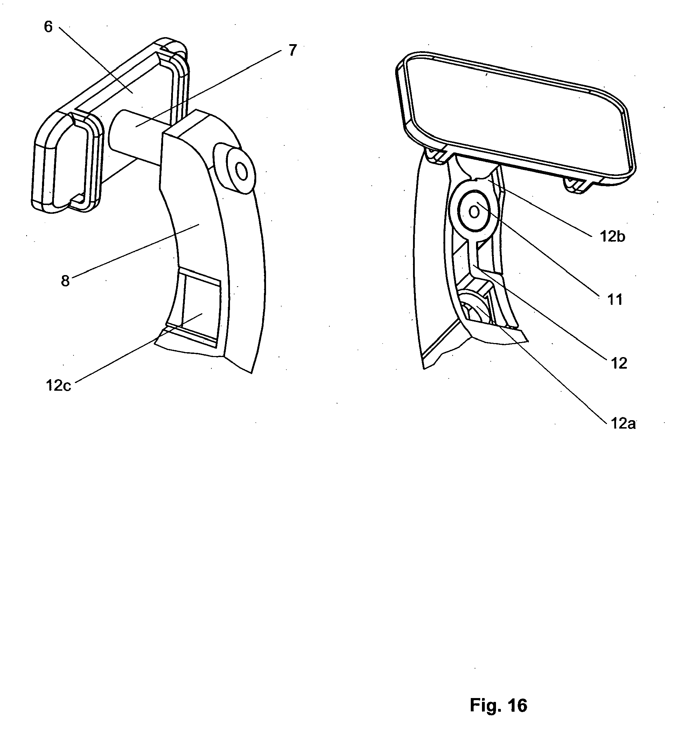

Fig. 9: Perspektivansichten einer Maske in zwei Stellbereichen mit Längsschnitten,
Fig. 9.1: eine Perspektiv- und Seitenansicht einer Maske,

Unter einer gestreckten Bewegung sind Bewegungen zu verstehen, die linear oder in einem großen Bogen erfolgen. Dabei kann der Bogen einer Kreisbahn oder einer beliebigen Bahn entsprechen. Eine gestreckte Bewegung liegt insbesondere dann vor, wenn der Radius der Bewegung zumindest eines Punktes des Stellgliedes zumindest auf einem Teilsegment der Bewegungsbahn größer als etwa 25 mm ist. In einer bevorzugten Ausführungsform ist der Radius der Bewegung zumindest eines Punktes des Stellgliedes zumindest auf einem Teilsegment der Bewegungsbahn größer als etwa 50 mm. Besonders bevorzugt ist ein Radius der Bewegung zumindest eines Punktes des Stellgliedes zumindest auf einem Teilsegment der Bewegungsbahn größer als etwa 75 mm.

Eine gestreckte Bewegung liegt auch dann vor, wenn die Bewegung eines Stellgliedes eine Überlagerung mehrerer Bewegungsformen ist. So entspricht das Abwälzen eines Zahnrades auf einer linearen oder gebogenen Kontur einer Rotation um die (ggf. virtuelle und ortsbewegliche) Zahnradachse bei gleichzeitigem Verschieben des Zahnrades entlang der Bahn der Wälzpartner. In diesem Fall kann die (ggf. virtuelle und ortsbewegliche) Achse des zahnrades eine gestreckte Bewegung im Sinne der oben stehenden Definition ausführen. Eine (ggf. virtuelle und ortsbewegliche) Achse ist ein Teil des Stellgliedes und kann somit eine gestreckte Bewegung im Sinne der oben stehenden Definition ausführen.

Weiterhin liegt eine gestreckte Bewegung vor, wenn ein Punkt des Stellgliedes eine gestreckte Bewegung im Sinne der oben stehenden Definition ausführt, während das Stellglied selbst eine beliebige komplexe Bewegung ausführen kann.

In bevorzugten Ausführungsformen erfolgt die Bewegung des Verstellelementes nicht proportional zur Bewegung, die die Stirnauflage vollzieht. Dieses erfolgt zumindest in Teilbereichen des Stellweges und lässt sich auf alle in dieser Schrift angeführten Ausführungsformen anwenden. Durch die nicht proportionale Übertragung der Bewegung wird z.B. im mittleren Stellbereich eine feinere Einstellung als in dem äußeren Stellbereichen ermöglicht.

Eine Ausführungsalternative für die Stirnstützenverstellung wird in der Fig.9 dargestellt. Bei dieser Stirnstützenverstellung besteht der Stirnstützenschaft (8) aus zwei Teilen, einem feststehenden (8a), mit dem Maskenkörper (1) verbundenen und einem beweglichen Teil (8b). Das feststehende Teil (8a) des Stirnstützenschaftes weist eine Öffnung (9) auf, in der der Arm des Stirnstützenträgers (7) aufgenommen wird. Der Arm des Stirnstützenträgers (7) besteht aus teleskopartig inund auseinander bewegbaren Teilen. Der bewegliche Teil des Stirnstützenschaftes (8b) wird am oberen Ende über eine Schnappverbindung (11) an einer Achse im Arm des Stirnstützenträgers mit dem teleskopartigen Arm des Stirnstützenträgers (7) verbunden. Das untere Ende des beweglichen Stirnstützenschaftes (8b) wird über zwei Halteelement (Pins) (11a) mit dem feststehenden Teil (8a) drehbar verbunden und ist so ausgeführt, dass er sich in das feststehende Teil (8a) einfügen kann. Des Weiteren weist der bewegliche Teil (8b) des Stirnstützenschaftes Führungsnuten (10) auf, in denen Führungselemente (Pins) eines Schiebers (5b) greifen. Der Schieber (5b) dient als Verstellelement und bewegt sich auf der Kontur des feststehenden Teiles (8ä) des Stirnstützenschaftes in einer gestreckten Bewegung. Durch Bewegen des Verstellelementes (Schiebers) (5b) wird der bewegliche Teil (8b) des Stirnstützenschaftes im feststehenden Teil (8a) bewegt und nimmt den teleskopartigen Arm des Stirnstützenträgers (7) dabei mit. Die Stirnstütze (6) wird dabei horizontal linear vor- und zurückgestellt.

Bevorzugt ist eine mechanische Betätigung des Teleskops, möglich ist aber auch eine hydraulische, pneumatische, etc. Betätigung.

Eine etwas einfachere Ausführungsalternative dieser Stirnstützenverstellung wird in Fig.9.1 dargestellt. Hierbei handelt es sich um eine zweigeteilte Stirnstützenverstellung, die einen Stirnstützenschaft (8) aufweist, der fest mit dem Maskenkörper (1) verbundenen ist und einem beweglichen Stirnstützenträgerarm (7), der sich beim Verstellen der Stirnstütze (6) in dem Stirnstützenschaft (8) bewegt. Die Verbindung zwischen Stirnstützenschaft (8) und Stirnstützenträgerarm (7) erfolgt über zwei Halteelemente (Pins) (11a), die am Stirnstützenträgerarm (7) angeordnet sind und drehbar im Stirnstützenschaft (8) gelagert sind. Der Stirnstützenträgerarm (7) ist schmaler als der Schaft (8) und bewegt sich in diesem vor und zurück.

Das Verstellen erfolgt über das Bewegen in einer gestreckten Bewegung eines Schiebers (5b), der über dem feststehenden Stirnstützenschaft (8) greift und Führungselemente (Pins) aufweist, die in Führungsnuten (10) im Stirnstützenträgerarm (7) greifen und diesen beim Verstellen bewegen.

Die Führungsnuten (10) im Stirnstützenträgerarm (7) laufen nach oben hin leicht schräg aus und bewirken bei leichtem Druckaufwand auf den Schieber (5b) ein Aufspreizen des Schiebers (5b) wodurch dieser sich leicht demontieren lässt. Danach lassen sich die Halteelemente (11a) des beweglichen Stirnstützenträgersarms (7) aus seiner unteren Halterung demontieren.

Zur Montage werden die Halteelemente (11a) des Stirnstützenträgerarms (7) in die Gegenlager im Stirnstützenschaft (8) eingeklickt, der Schieber (5b) kann danach leicht von oben in die Führungsnuten (10) einführen werden oder aber die Führungselemente (Pins) des Schiebers (5b) lassen sich durch die Schwenkbewegung des Stirnstützenträgerarms (7) aufweiten und schnappen so wieder in die Führungsnuten (10) ein.

Erfindungsgemäß ist auch an eine Kennzeichnung (17) der eingestellten Position des Abstützelementes (6) bzw. des Stellgliedes (5b) gedacht. So kann z.B. eine spezielle Einstell-Position, ein Einstell-Bereich oder eine Mittelstellung des Stellbereiches gekennzeichnet werden oder auch jede Raststellung. Dieses kann über eine optische und/oder akustische und/oder taktile Kennzeichnung (17) der eingestellten Position des Abstützelements bzw. des Stellglieds erfolgen. Es können auch mehrere Positionen oder Positionsbereiche im Stellbereich der Vorrichtung zum Abstützen, optisch und/oder akustisch und/oder taktil hervorgehoben sein.

## Patentansprüche

1. Vorrichtung zur Beatmung, zumindest bestehend aus einem Maskenkörper (1), welcher einen Innenraum von einem Aussenbereich abgrenzt, einem Bereich zur Abdichtung (2) der Vorrichtung gegen Gesichtspartien eines Patienten, Befestigungselementen (3) zur Positionierung der Vorrichtung am Kopf des Patienten und einem Anschluss zur Zufuhr von Atemgas (4), sowie einer der Abstützung an der Stirn dienende Stirnstütze (6), die einen Stirnstützenträgerarm (7) aufweist, wobei der Stirnstützenträgerarm (7) Führungsnuten (10) aufweist, wobei der Maskenkörper (1) einen fest verbundenen Stirnstützenschaft (8) aufweist, wobei die Verbindung zwischen Stirnstützenschaft (8) und Stirnstützenträgerarm (7) über zwei Halteelemente (11a) erfolgt, die am Stirnstützenträgerarm (7) angeordnet und drehbar im Stirnstützenschaft (8) gelagert sind und wobei der Stirnstützenträgerarm (7) schmaler als der Stirnstützenschäft (8) ist **dadurch gekennzeichnet, dass** das Verstellen über das Bewegen eines als Schieber (5b) ausgebildeten Stellgliedes, in einer gestreckten Bewegung, erfolgt, wobei der Schieber (5b) über den feststehenden Stirnstützenschaft (8) greift und wobei der Schieber Führungselemente aufweist, die in die Führungsnuten (10) im Stirnstützenträgerarm (7) greifen und diesen beim Verstellen vor und zurück bewegen.

2. Vorrichtung nach Anspruch 1, **dadurch gekennzeichnet, dass** die Führungsnuten (10) einen gebogenen Verlauf aufweisen.

3. Vorrichtung nach Anspruch 1 oder 2, **dadurch gekennzeichnet, dass** die Bewegung der Stirnstütze (6) zumindest bereichsweise in einem Winkel grösser als 0[deg.] zur Bewegungsrichtung des Stellgliedes (5b) erfolgt.

4. Vorrichtung nach einem der vorhergehenden Ansprüche, **dadurch gekennzeichnet, dass** die Bewegung der Stirnstütze (6) zumindest in Teilen des Stellbereichs nicht proportional zur Bewegung des Stellgliedes (5b) ist.

5. Vorrichtung nach einem der vorstehenden Ansprüche, **dadurch gekennzeichnet, dass** die Vorrichtung über eine optische und/oder akustische und/oder taktile Kennzeichnung (17) der eingestellten Position der Stirnstütze (6) beziehungsweise des Stellgliedes (5a, 5b) verfügt.

6. Vorrichtung nach Anspruch 5, **dadurch gekennzeichnet, dass** eine spezielle Einstell-Position oder ein spezieller Einstellbereich der Stirnstütze (6), nämlich eine Position oder ein Einstellbereich im mittleren Bereich des Stellbereichs der Stirnstütze (6), optisch und/oder akustisch und/oder taktil hervorgehoben ist.

7. Vorrichtung nach Anspruch 5 oder 6, **dadurch gekennzeichnet, dass** mehrere Positionen oder Positionsbereiche im Stellbereich der Stirnstütze (6), optisch und/oder akustisch und/oder taktil hervorgehoben sind.

8. Vorrichtung nach einem der vorstehenden Ansprüche, **dadurch gekennzeichnet, dass** das Verstellen des Stellgliedes (5b) in einer gerasteten Bewegung erfolgt.

9. Vorrichtung nach einem der vorstehenden Ansprüche, **dadurch gekennzeichnet, dass** das Stellglied (5b) beim Verstellen eine im wesentlichen rotatorische Bewegung ausführt.

## Claims

1. Device for ventilation, consisting of at least a mask body (1) that separates an interior space from an exterior space, a section for sealing (2) the device against parts of a patient's face, fastening elements (3) for positioning the device on the patient's head, and a connection for the supply of respiratory gas (4), as well as a forehead support (6) used to rest the device against the patient's forehead, featuring a forehead support bearing arm (7), whereby the support bearing arm (7) has guide grooves (10) and the mask body (1) has a permanently attached forehead support shaft (8), wherein the forehead support shaft (8) and the forehead support bearing arm (7) are connected by two retaining elements (11a) arranged on the forehead support bearing arm (7) and pivoted in the forehead support shaft (8) and where the forehead support bearing arm (7) is narrower than the forehead support shaft (8), **characterized by** the fact that the adjustment is performed by moving an adjusting element in the form of a slider (5b) in an extended movement, wherein the slider (5b) reaches over the stationary forehead support shaft (8) and wherein the slider has guide elements that reach into the guide grooves (10) in the forehead support bearing arm (7), moving it forwards and backwards when it is adjusted.

2. Device according to claim 1 **characterized by** the fact that the guide grooves (10) follow a curving path.

3. Device according to claim 1 or 2 **characterized by** the fact that the forehead support (6) moves at an angle greater than 0[deg] in the direction of movement of the adjusting element (5b) at least in certain areas.

4. Device according to one of the above claims **characterized by** the fact that the movement of the forehead support (6) is not proportional to the movement of the adjusting element (5b), at least in parts of the adjustment range.

5. Device according to one of the above claims characterized by- the fact that the device has a visible and/or audible and/or tactile indication (17) of the set position of the forehead support (6) or of the adjusting element (5a, 5b).

6. Device according to claim 5 **characterized by** the fact that a special adjustment position or a special adjustment range of the forehead support (6), specifically a position or adjustment range within the middle area of the setting range of the forehead support (6) is accented visually and/or audibly and/or by touch.

7. Device according to claim 5 or 6 **characterized by** the fact that several positions or position ranges within the adjustment range of the forehead support (6) are accented visually and/or audibly and/or by touch.

8. Device according to one of the above claims **characterized by** the fact that the adjusting element (5b) moves in fixed increments.

9. Device according to one of the above claims **characterized by** the fact that the adjusting element (5b) executes a primarily rotational movement when being adjusted.

## Revendications

1. Dispositif de ventilation composé au minimum d'une coque de masque (1) qui sépare une zone intérieure d'une zone extérieure, d'une zone destinée à assurer l'étanchéité (2) du dispositif par rapport aux parties du visage d'un patient, d'éléments de fixation (3) pour le positionnement du dispositif sur la tête du patient et d'un raccord pour l'alimentation en gaz respiratoire (4), ainsi que d'une cale frontale (6) servant d'appui pour le front et dotée d'un bras de support frontal (7), lequel bras de support frontal (7) dispose de rainures de guidage (10), la coque du masque (1) étant dotée d'un axe de support frontal fixe (8), la liaison entre l'axe de support frontal (8) et le bras de support frontal (7) étant assurée par deux éléments de fixation (11a) fixés sur le bras de support frontal (7) et logés dans l'axe de support frontal (8) dans lequel ils tournent et le bras de support frontal (7) étant plus étroit que l'axe de support frontal (8), **caractérisé en ce que** le réglage est effectué par un mouvement d'étirement d'un élément de réglage de la forme d'un curseur (5b), ledit curseur (5b) étant en prise sur l'axe de support frontal fixe (8) et doté d'éléments de guidage évoluant dans les rainures de guidage (10) du bras de support frontal (7) et qui avancent et reculent ce bras lors du réglage.

2. Dispositif selon la revendication 1 **caractérisé en ce que** les rainures de guidage (10) présentent un profil incurvé.

3. Dispositif selon la revendication 1 ou 2 **caractérisé en ce que** le mouvement de la cale frontale (6) s'effectue au moins par zones dans un angle supérieur à 0[deg.] par rapport à la direction du mouvement de l'élément de réglage (5b).

4. Dispositif selon les revendications précédentes **caractérisé en ce que** le mouvement de la cale frontale (6) n'est pas proportionnel au mouvement de l'élément de réglage (5b) dans au moins des parties de la plage de réglage.

5. Dispositif selon les revendications précédentes **caractérisé en ce que** le dispositif dispose d'un repérage visuel et/ou sonore et/ou tactile (17) de la position définie de la cale frontale (6) et de l'élément de guidage (5a, 5b).

6. Dispositif selon la revendication 5 **caractérisé en ce qu'**une position de réglage spéciale ou plage de réglage spéciale de la cale frontale (6), à savoir une position ou une plage de réglage dans la zone médiane de la plage de réglage de la cale frontale (6), est indiquée de manière visuelle et/ou sonore et/ou tactile.

7. Dispositif selon la revendication 5 ou 6 **caractérisé en ce que** plusieurs positions ou plages de positions au sein de la plage de réglage de la cale frontale (6) sont indiquées de manière visuelle et/ou sonore et/ou tactile.

8. Dispositif selon les revendications précédentes **caractérisé en ce que** le réglage de l'élément de réglage (5b) s'effectue dans un mouvement à encliquetage.

9. Dispositif selon les revendications précédentes **caractérisé en ce que** l'élément de réglage (5b) exécute essentiellement un mouvement de rotation lors du réglage.
